# EUROPEAN PATENT APPLICATION

(11) **EP 4 137 105 A1**
(43) Date of publication of application: **22.02.2023**
(21) Application number: 22163402.5
(22) Date of filing: 22.03.2022
(51) Int. Cl.: A61F 13/00, A61F 13/08, B32B 25/10

(54) **RUBBER STRIP COMPRESSION GARMENT FABRIC AND PREPARATION METHOD THEREOF**

(30) Priority: 18.08.2021 CN 202110948005
(71) Applicant: Xun, Wei, Xi'an, Shaanxi (CN)
(72) Inventor: Xun, Wei, Xi'an, Shaanxi (CN)
(74) Representative: Cleanthous, Marinos

(57) **Abstract**

The present invention discloses a rubber strip compression garment fabric and a preparation method thereof, and relates to the technical field of compression garment fabrics. The rubber strip compression garment fabric is compounded by an antibacterial and mite-proof elastic fabric and rubber strips. The antibacterial and mite-proof elastic fabric includes the following raw material components in percentage by weight: 75% of nylon fiber, 18% of lycra fiber, 2% of carbon fiber and 5% of antibacterial and mite-proof PET fiber; the antibacterial and mite-proof PET fiber includes the following raw material components in percentage by weight: 1-5% of an antibacterial and mite-proof agent and 95-99% of PET. The rubber strip compression garment fabric provided by the present invention is breathable, quick-drying, moisture-absorbing and sweat-releasing, strong in wear resistance, and antibacterial and mite-proof, and can make limbs flexibly stretch and provide strong supporting pressure.

## Description

### Technical Field

The present invention relates to the technical field of compression garment fabrics, in particular to a rubber strip compression garment fabric and a preparation method thereof.

### Background

Fitness is a common sport item, including various sports forms, such as swimming, brisk walking, jogging, cycling and other aerobic sports, as well as exercises with weightlifting equipment such as dumbbells, barbells and kettles. In the process of exercise, a human body will sweat a lot which may pollute fitness clothes. If the fitness clothes are not cleaned in time, breeding of bacteria and mites will happen easily. In addition, during force exertion in the process of exercise, failure to make actions in place or exert force correctly will lead to muscle injuries.

At present, there is no fabric on the market, which is breathable, quick-drying, moisture-absorbing and sweat-releasing, strong in wear resistance, and antibacterial and mite-proof and can make limbs flexibly stretch and provide strong supporting pressure.

### Summary

Aiming at defects in the prior art, the present invention provides a rubber strip compression garment fabric and a preparation method thereof.

The rubber strip compression garment fabric is vertically compounded by an antibacterial and mite-proof elastic fabric and rubber strips, wherein the antibacterial and mite-proof elastic fabric includes the following raw material components in percentage by weight: 75% of nylon fiber, 18% of lycra fiber, 2% of carbon fiber and 5% of antibacterial and mite-proof PET fiber; the antibacterial and mite-proof PET fiber includes the following raw material components in percentage by weight: 1-5% of an antibacterial and mite-proof agent and 95-99% of PET; and the antibacterial and mite-proof agent includes the following raw materials: chitin and tea saponin.

Preferably, the antibacterial and mite-proof PET fiber includes the following raw material components in percentage by weight: 3% of an antibacterial and mite-proof agent and 97% of PET.

Preferably, the antibacterial and mite-proof PET fiber includes the following raw material components in percentage by weight: 4% of an antibacterial and mite-proof agent and 96% of PET.

Preferably, the antibacterial and mite-proof PET fiber includes the following raw material components in percentage by weight: 2% of an antibacterial and mite-proof agent and 98% of PET.

Preferably, a weight part ratio of chitin to tea saponin is 3: 2.

The preparation method of the rubber strip compression garment fabric includes the following steps: S1, uniformly mixing chitin and tea saponin to prepare an antibacterial and mite-proof agent; S2, adding the antibacterial and mite-proof agent into PET, and performing melt spinning and drafting to prepare antibacterial and mite-proof PET fiber; S3, blending the antibacterial and mite-proof PET fiber and lycra fiber to prepare warps; S4, pre-stretching nylon fiber, and blending with carbon fiber to prepare wefts; S5, circularly weaving the warps and the wefts to prepare an antibacterial and mite-proof elastic fabric; and S6, compounding the antibacterial and mite-proof elastic fabric and a rubber layer to prepare the rubber strip compression garment fabric.

Preferably, in step S2, the temperature of melt spinning is 270-280°C and the duration is 45 min.

Preferably, in step S2, the number of drafting multiples of the drafting is 3.5-4, drafting speed is 1000 m/min, and drafting temperature is 210-220°C.

Preferably, in step S4, the number of pre-stretching multiples is 3-5.

The present invention has the beneficial effects that:

(1) The rubber strip compression garment fabric provided by the present invention is breathable, quick-drying, moisture-absorbing and sweat-releasing, strong in wear resistance, and antibacterial and mite-proof, and can make limbs flexibly stretch and provide strong supporting pressure.

(2) In the preparation method of the rubber strip compression garment fabric provided by the present invention, circular weaving is adopted, so that the prepared fabric has equivalent tension and compressibility in all directions, and the fabric can make the limbs flexibly stretch and provide the strong supporting pressure; the elastic fabric is compounded with the rubber strips, which can stabilize muscles around and compress strongly, so as to prevent muscle strain, while protection of the rubber strips for muscles and joints will be stronger, more accurate and more effective. The fabric can make the limbs flexibly stretch and provide the strong supporting pressure and is breathable, quick-drying, moisture-absorbing and sweat-releasing, and strong in wear resistance.

### Detailed Description

Embodiments of the technical solutions of the present invention will be described below in detail. The following embodiments are only used for more clearly illustrating the technical solutions of the present invention, and thus are only used as examples, not used for limiting the protection scope of the present invention.

It should be noted that unless otherwise stated, technical terms or scientific terms used in the present application should have the ordinary meanings generally understood by those skilled in the field to which the present invention belongs.

Embodiment 1

A rubber strip compression garment fabric is provided. The rubber strip compression garment fabric is vertically compounded by an antibacterial and mite-proof elastic fabric and rubber strips. The antibacterial and mite-proof elastic fabric includes the following raw material components in percentage by weight: 75% of nylon fiber, 18% of lycra fiber, 2% of carbon fiber and 5% of antibacterial and mite-proof PET fiber; the antibacterial and mite-proof PET fiber includes the following raw material components in percentage by weight: 3% of an antibacterial and mite-proof agent and 97% of PET; and a weight part ratio of chitin to tea saponin is 3: 2.

A preparation method of the rubber strip compression garment fabric is characterized in that:

The preparation method includes the following steps:

S1, uniformly mixing chitin and tea saponin to prepare an antibacterial and mite-proof agent;

S2, adding the antibacterial and mite-proof agent into PET, and performing melt spinning and drafting to prepare antibacterial and mite-proof PET fiber;

S3, blending the antibacterial and mite-proof PET fiber and lycra fiber to prepare warps;

S4, pre-stretching nylon fiber, and blending with carbon fiber to prepare wefts;

S5, circularly weaving the warps and the wefts to prepare an antibacterial and mite-proof elastic fabric; and

S6, compounding the antibacterial and mite-proof elastic fabric and a rubber layer to prepare the rubber strip compression garment fabric.

In step S2, the temperature of melt spinning is 275 °C and the duration is 45 min.

In step S2, the number of drafting multiples of the drafting is 3.5, drafting speed is 1000 m/min, and drafting temperature is 215°C.

In step S4, the number of pre-stretching multiples is 4.

Embodiment 2

A rubber strip compression garment fabric is provided. The rubber strip compression garment fabric is vertically compounded by an antibacterial and mite-proof elastic fabric and rubber strips. The antibacterial and mite-proof elastic fabric includes the following raw material components in percentage by weight: 75% of nylon fiber, 18% of lycra fiber, 2% of carbon fiber and 5% of antibacterial and mite-proof PET fiber; the antibacterial and mite-proof PET fiber includes the following raw material components in percentage by weight: 2% of an antibacterial and mite-proof agent and 98% of PET; and a weight part ratio of chitin to tea saponin is 3: 2.

A preparation method of the rubber strip compression garment fabric is characterized in that:

The preparation method includes the following steps:

S1, uniformly mixing chitin and tea saponin to prepare an antibacterial and mite-proof agent;

S2, adding the antibacterial and mite-proof agent into PET, and performing melt spinning and drafting to prepare antibacterial and mite-proof PET fiber;

S3, blending the antibacterial and mite-proof PET fiber and lycra fiber to prepare warps;

S4, pre-stretching nylon fiber, and blending with carbon fiber to prepare wefts;

S5, circularly weaving the warps and the wefts to prepare an antibacterial and mite-proof elastic fabric; and

S6, compounding the antibacterial and mite-proof elastic fabric and a rubber layer to prepare the rubber strip compression garment fabric.

In step S2, the temperature of melt spinning is 275 °C and the duration is 45 min.

In step S2, the number of drafting multiples of the drafting is 3.5, drafting speed is 1000 m/min, and drafting temperature is 215°C.

In step S4, the number of pre-stretching multiples is 4.

Embodiment 3

A rubber strip compression garment fabric is provided. The rubber strip compression garment fabric is vertically compounded by an antibacterial and mite-proof elastic fabric and rubber strips. The antibacterial and mite-proof elastic fabric includes the following raw material components in percentage by weight: 75% of nylon fiber, 18% of lycra fiber, 2% of carbon fiber and 5% of antibacterial and mite-proof PET fiber; the antibacterial and mite-proof PET fiber includes the following raw material components in percentage by weight: 4% of an antibacterial and mite-proof agent and 96% of PET; and a weight part ratio of chitin to tea saponin is 3: 2.

A preparation method of the rubber strip compression garment fabric is characterized in that:

The preparation method includes the following steps:

S1, uniformly mixing chitin and tea saponin to prepare an antibacterial and mite-proof agent;

S2, adding the antibacterial and mite-proof agent into PET, and performing melt spinning and drafting to prepare antibacterial and mite-proof PET fiber;

S3, blending the antibacterial and mite-proof PET fiber and lycra fiber to prepare warps;

S4, pre-stretching nylon fiber, and blending with carbon fiber to prepare wefts;

S5, circularly weaving the warps and the wefts to prepare an antibacterial and mite-proof elastic fabric; and

S6, compounding the antibacterial and mite-proof elastic fabric and a rubber layer to prepare the rubber strip compression garment fabric.

In step S2, the temperature of melt spinning is 275 °C and the duration is 45 min.

In step S2, the number of drafting multiples of the drafting is 3.5, drafting speed is 1000 m/min, and drafting temperature is 215°C.

In step S4, the number of pre-stretching multiples is 4.

Test Example 1

Antibacterial properties of the rubber strip compression garment fabrics prepared in embodiments 1-3 of the present invention are tested, and the results are shown in Table 1.

Test method: GB/T 20944.3-2008 *Textiles-Evaluation for antibacterial activity*

**Table 1**

| | Parameter | Required value | Measured value | Single judgment |
|---|---|---|---|---|
| Embodiment 1 | Antibacterial rate of *Escherichia coli* (8099)/% | ≥70 | 98 | Satisfied |
| | Antibacterial rate of *Staphylococcus aureus* (ATCC6538)/% | ≥70 | 98 | Satisfied |
| | Antibacterial rate of *Candida albicans* (ATCC10231)/% | ≥60 | 90 | Satisfied |
| Embodiment 2 | Antibacterial rate of *Escherichia coli* (8099)/% | ≥70 | 97 | Satisfied |
| | Antibacterial rate of *Staphylococcus aureus* (ATCC6538)/% | ≥70 | 97 | Satisfied |
| | Antibacterial rate of *Candida albicans* (ATCC10231)/% | ≥60 | 89 | Satisfied |
| Embodiment 3 | Antibacterial rate of *Escherichia coli* (8099)/% | ≥70 | 99 | Satisfied |
| | Antibacterial rate of *Staphylococcus aureus* (ATCC6538)/% | ≥70 | 99 | Satisfied |
| | Antibacterial rate of *Candida albicans* (ATCC10231)/% | ≥60 | 91 | Satisfied |

Test Example 2

Antibacterial properties of the rubber strip compression garment fabrics prepared in embodiments 1-3 of the present invention are tested, and the results are shown in Table 2.

Test method: GB/T 24253-2009 *Textiles-Evaluation for anti-mites activity*

**Table 2**

| | Removal rate (%) | Mite-proof effect |
|---|---|---|
| Embodiment 1 | 96 | Very strong |
| Embodiment 2 | 95 | Very strong |
| Embodiment 3 | 97 | Very strong |

Finally, it should be noted that the above embodiments are only used for describing the technical solutions of the present invention rather than limiting the present invention. Although the present invention is described in detail by referring to the above embodiments, those ordinary skilled in the art should understand that the technical solution recorded in each of the above embodiments can be still amended, or some or all technical features therein can be replaced equivalently. However, the changes or replacements do not make the essence of the corresponding technical solution depart from the scope of the technical solutions of embodiments of the present invention, and shall be covered within the scope of the claims and the description of the present invention.

## Claims

1. A rubber strip compression garment fabric, which is vertically compounded by an antibacterial and mite-proof elastic fabric and rubber strips, wherein the antibacterial and mite-proof elastic fabric comprises the following raw material components in percentage by weight: 75% of nylon fiber, 18% of lycra fiber, 2% of carbon fiber and 5% of antibacterial and mite-proof PET fiber; the antibacterial and mite-proof PET fiber comprises the following raw material components in percentage by weight: 1-5% of an antibacterial and mite-proof agent and 95-99% of PET; and the antibacterial and mite-proof agent comprises the following raw materials: chitin and tea saponin.

2. The rubber strip compression garment fabric according to claim 1, wherein the antibacterial and mite-proof PET fiber comprises the following raw material components in percentage by weight: 3% of an antibacterial and mite-proof agent and 97% of PET.

3. The rubber strip compression garment fabric according to claim 1, wherein the antibacterial and mite-proof PET fiber comprises the following raw material components in percentage by weight: 4% of an antibacterial and mite-proof agent and 96% of PET.

4. The rubber strip compression garment fabric according to claim 1, wherein the antibacterial and mite-proof PET fiber comprises the following raw material components in percentage by weight: 2% of an antibacterial and mite-proof agent and 98% of PET.

5. The rubber strip compression garment fabric according to claim 1, wherein a weight part ratio of chitin to tea saponin is 3: 2.

6. A preparation method of the rubber strip compression garment fabric of claim 1, comprising the following steps: S1, uniformly mixing chitin and tea saponin to prepare an antibacterial and mite-proof agent; S2, adding the antibacterial and mite-proof agent into PET, and performing melt spinning and drafting to prepare antibacterial and mite-proof PET fiber; S3, blending the antibacterial and mite-proof PET fiber and lycra fiber to prepare warps; S4, pre-stretching nylon fiber, and blending with carbon fiber to prepare wefts; S5, circularly weaving the warps and the wefts to prepare an antibacterial and mite-proof elastic fabric; and S6, compounding the antibacterial and mite-proof elastic fabric and a rubber layer to prepare the rubber strip compression garment fabric.

7. The preparation method of the rubber strip compression garment fabric according to claim 6, wherein in step S2, the temperature of melt spinning is 270-280°C and the duration is 45 min.

8. The preparation method of the rubber strip compression garment fabric according to claim 6, wherein in step S2, the number of drafting multiples of the drafting is 3.5-4, drafting speed is 1000 m/min, and drafting temperature is 210-220°C.

9. The preparation method of the rubber strip compression garment fabric according to claim 6, wherein in step S4, the number of pre-stretching multiples is 3-5.
